Europäisches Patentamt

European Patent Office

Office européen des brevets

(11) Veröffentlichungsnummer: **0 382 129 B1**

# (12) EUROPÄISCHE PATENTSCHRIFT

(45) Veröffentlichungstag der Patentschrift: **13.10.93**

(21) Anmeldenummer: **90102174.1**

(22) Anmeldetag: **03.02.90**

(51) Int. Cl.⁵: **A61M 35/00, A61M 31/00**

(54) Therapeutisches System zur transdermalen oder transmucosalen Verabreichung von Wirkstoffen und seine Verwendung.

(30) Priorität: **09.02.89 DE 3903794**

(43) Veröffentlichungstag der Anmeldung:
**16.08.90 Patentblatt 90/33**

(45) Bekanntmachung des Hinweises auf die Patenterteilung:
**13.10.93 Patentblatt 93/41**

(84) Benannte Vertragsstaaten:
**AT BE CH DE DK ES FR GB GR IT LI LU NL SE**

(56) Entgegenhaltungen:
**EP-A- 0 241 119**
**EP-A- 0 249 475**
**FR-A- 2 562 800**
**US-A- 4 764 379**

(73) Patentinhaber: **LTS Lohmann Therapie-
Systeme GmbH & Co. KG
Postfach 23 43
D-56513 Neuwied(DE)**

(72) Erfinder: **Müller, Walter, Dr., Dipl.-Chem.
Engerser Strasse 56
D-5450 Neuwied 1(DE)**
Erfinder: **Roreger, Michael
Otto-Hahn-Strasse 16
D-5450 Neuwied 22(DE)**
Erfinder: **Kindel, Heinrich
Westerwaldstrasse 7
D-5455 Rengsdorf(DE)**
Erfinder: **Bohnenkämper, Peter
Eschenweg 15
D-4788 Warstein 1(DE)**
Erfinder: **Palm, Heike
Obere Grabenstrasse 24
D-5405 Ochtendung(DE)**

(74) Vertreter: **Flaccus, Rolf-Dieter, Dr.
Patentanwalt
Sperlingsweg 32
D-50389 Wesseling (DE)**

Rank Xerox (UK) Business Services
(3. 10/3.6/3.3. 1)

**Beschreibung**

Die Erfindung betrifft ein therapeutisches System beziehungswiese ein Verfahren zur Herstellung eines therapeutischen Systems zur transdermalen oder transmucosalen Verabreichung von Wirkstoffen aus einer undurchlässigen Rückschicht, einem zumindest zum Teil wirkstoffhaltigen zweischichtigen, aus voneinander getrennten Teilen bestehenden, Reservoir und einer undurchlässigen und ablösbaren Schutzschicht.

Transdermale Systeme haben sich durch die ihnen eigenen Vorteile mittlerweile in der Medizin ihren festen Platz erobert. Es sind mittlerweile eine ganze Reihe von verschieden aufgebauten Systemen in der Literatur beschrieben worden oder schon auf dem Markt. So ist in dem U.S.-Patent 3,734,097 (Alza) ein einfaches einschichtiges System und in dem U.S.-Patent 3,598,122 des gleichen Anmelders ein komplizierter aufgebautes mehrschichtiges System, bei dem zusätzliche Membranen Steuerfunktionen übernehmen, beschrieben.

Bei einem aus dem U.S.-Patent 4,764,379 bekannten therapeutischen System zur transdermalen Verabreichung von Wirkstoffen weist dieses einen ebenfalls sehr komplizierten, vielschichtigen Aufbau auf, umfassend eine undurchlässige Rückschicht, ein den Wirkstoff enthaltendes Reservoir, weiterhin zwei Penetrationsbeschleuniger enthaltende Schichten, wobei zwischen dem Wirkstoffreservoir und einer Penetrationsbeschleuniger enthaltenden Schicht eine Kontrollmembran angeordnet sein kann, sowie schließlich eine abziehbare Schutzschicht. Eine der Penetrationsbeschleuniger enthaltenden Schichten kann zur Befestigung des Systems auf der Haut selbstklebend ausgerüstet sein. Während des Zeitintervalls zwischen Herstellung und Anwendung kann die Selbstklebeschicht sowohl Wirkstoff als auch Penetrationsbeschleuniger absorbieren, und zwar in Abhängigkeit sowohl von der Schichtdicke als auch von der Länge des Zeitintervalls. Bei genügend langer Absorptionszeit führt dieser Vorgang zu einer Sättigung der Schicht mit Wirkstoff und Beschleuniger.

Unter therapeutischen Systemen sind arzneistoffhaltige Vorrichtungen bzw. Darreichungsformen zu verstehen, die einen Arzneistoff oder mehrere in vorausbestimmter Rate über einen festgelegten Zeitraum an einen festgelegten Anwendungsort abgeben (siehe Heilmann, "Therapeutische Systeme", Enke Verlag, Stuttgart, 1984, Seite 24). Ein transdermales therapeutisches System gibt den Wirkstoff über die Haut ab und wird demgemäß auf der Haut, also topisch angewandt.

Erst in jüngerer Zeit wurden Systeme beschrieben, bei denen vor Applikation spezielle Manipulationen durchzuführen sind. Man kann diese Systeme in zwei Kategorien einteilen. Zur ersten Kategorie zählen Systeme, bei denen der Wirkstoff überführt wird. Dies ist zum Beispiel notwendig, wenn der Wirkstoff in seiner bioverfügbaren Form instabil ist. Ein Beispiel für diese Kategorie sind die in der EP-A 0252459 (Schering Corporation) und in der Veröffentlichung von C.D. Ebert beschriebenen Mehrkammersysteme. Die Kammern liegen dabei nebeneinander in der gleichen Ebene und die vor Applikation durchzuführende Manipulation besteht darin, daß die flüssigen Kammerinhalte - etwa durch das Zerbrechen der die Kammern trennenden Kammerwände - zusammengebracht werden.

Zur zweiten Kategorie gehören Systeme wie sie in der EP-A 0249475 (Alza) beschrieben sind. Hier wird eine wirkstofffreie Schicht erst unmittelbar vor Gebrauch auf ein wirkstoffhaltiges Reservoir aufgebracht und dann mit der anderen Seite dieser Schicht auf die Haut geklebt. Der Wirkstoff gelangt erst zur Haut, wenn er diese wirkstofffreie Schicht passiert hat. Daraus resultiert ein gezielter Verzögerungseffekt, der z.B. von Vorteil ist, wenn nach abendlicher Applikation erst in den frühen Morgenstunden Wirkstoffspiegel wünschenswert sind.

Insbesondere bei Systemen gemäß EP-A-0249475 sind die durchzuführenden Manipulationen recht kompliziert. Hier müssen zwei flächenförmige flexible Gebilde vollflächig miteinander in Kontakt gebracht werden, so daß ein Laminat entsteht. Dies wird zusätzlich dadurch erschwert, daß zumindest ein flächenförmiges Gebilde selbstklebend sein muß. Auch der vorgeschlagene Systemaufbau, bei dem Reservoir und Verzögerungsschicht durch eine Art Scharnier miteinander verbunden sind, macht die durchzuführende Manipulation zu einem relativ schwierig durchzuführenden, fehleranfälligen Prozeß. Da mittels transdermaler Systeme medizinische Wirkstoffe verabreicht werden, ist somit mit diesem Systemaufbau die Arzneimittelsicherheit nicht gewährleistet.

Aufgabe der Erfindung war es nun, auch für solche oder ähnlich gelagerte Fälle ein System mit verbesserter Arzneimittelsicherheit zu entwickeln, das es auch ungeübten Personen ermöglicht, die erforderlichen Manipulationen leicht durchzuführen und das ohne größeren Aufwand herstellbar ist.

Einzelheiten der Erfindung werden beispielhaft anhand der Figuren erläutert.

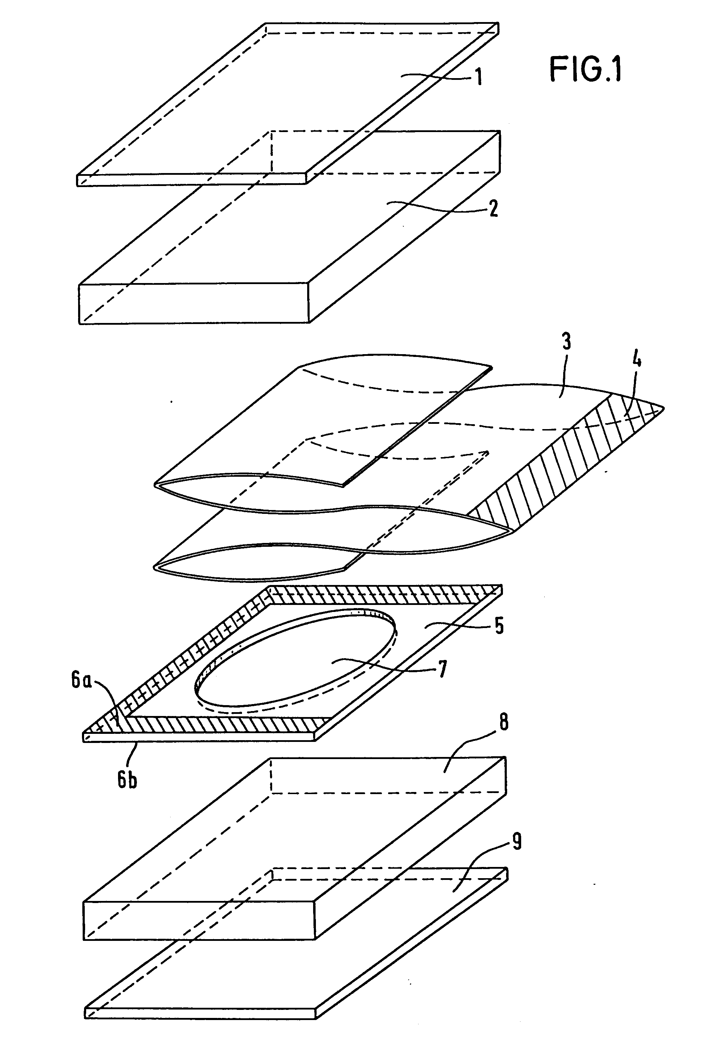

In Fig. 1 ist ein System gemäß Erfindung dargestellt. Es besteht aus der für die Inhaltsstoffe des oberen Reservoirteils (2) undurchlässigen Trägerfolie (1), dem oberen Reservoirteil (2), einer die Aussparung (7) der Lochmaske (5) abdeckenden und herausziehbaren Trennfolie (3) mit einem Anfaßstück (4), der Lochmaske (5) mit einer Aussparung (7), dem unteren Teil des Reservoirs (8) und einer für die Inhaltsstoffe des unteren Reservoirteils (8) undurchlässigen und vor Gebrauch zu entfernenden Abziehfolie (9).

Die Lochmaske (5) steht über ihre schraffiert gezeichnete Kontaktfläche (6a) mit dem oberen Reservoir und mit der Kontaktfläche (6b) mit dem unteren Reservoir (8) in Verbindung. Die Kontaktfläche (6b) entspricht somit der gesamten verfügbaren Oberfläche, d.h. der Rechtecksfläche der Lochmaske (5) abzüglich der Fläche der Aussparung (7). Die Lochmaske (5) ist selbstverständlich bis auf die Aussparung (7) ebenso wie die Trennfolie (3) für alle Inhaltsstoffe der beiden Reservoirteile undurchlässig. Bei dem gezeichneten Aufbau ist vorausgesetzt, daß die beiden vor Applikation getrennten Reservoirteile selbstklebend sind. Der Zusammenhalt des Systems wird dann dadurch gewährleistet, daß Reservoirteil (8) vollflächig auf der Kontaktfläche (6b) und Reservoirteil (2) auf der schraffierten Fläche (6a) der Lochmaske (5) kleben.

Sollten die betreffenden Flächen der getrennten Reservoirteile nicht selbstklebend sein, ist es keine Schwierigkeit, sie entweder vollflächig oder nur an den dafür relevanten Teilen mit einem zusätzlichen klebenden Film zu versehen. Gleiches gilt sinngemäß für die Kontaktfläche des oberen Reservoirteils mit der Trägerfolie (1) und der nach Applikation der Haut zugewandten Seite des unteren Reservoirteils (8).

Selbstverständlich kann die Aussparung (7) der Lochmaske (5) auch eine andere als kreisförmige Geometrie haben, und besonders zur Vergrößerung der Kontaktfläche zwischen den beiden zu vereinigenden Reservoirteilen ist eine an den Buchstaben "U" erinnernde Form geeignet.

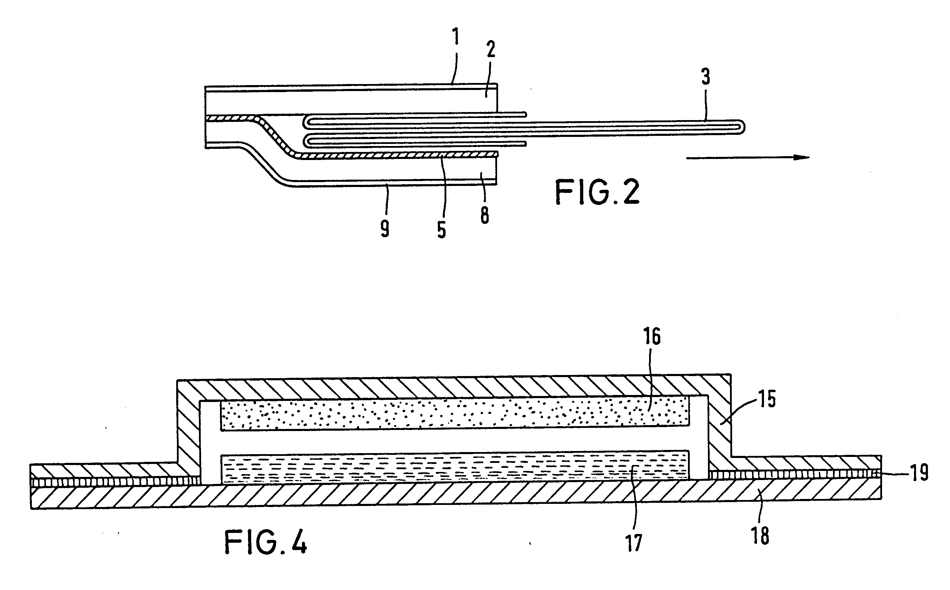

Aktiviert wird das System durch das Herausziehen der speziell gefalteten und silikonisierten Folie (3). Die spezielle Faltung erlaubt auch dann das leichte Herausziehen, wenn beide Reservoirteile selbstklebend sind, da durch sie die Kräfte so umgelenkt werden, daß der Kraftvektor senkrecht auf der Ebene der Reservoirteile steht. Dies wird in Fig. 2 verdeutlicht, die einen Blick auf die Schmalseite des in der Mitte durchgeschnittenen Systems zeigt. Eine ähnlich gefaltete Folie wird in der EP-A 0241119 (American Home Products Corporation) benutzt, um ein nur in einer aluminisierten Verpackung lagerbares wirkstoffhaltiges Reservoir unmittelbar vor Gebrauch mit einer nichtaluminisierten Trägerfolie für die transdermale Applikation zu versehen. Der Verwendungszweck ist hier also ein ganz anderer, und es wurde zudem nicht die Möglichkeit erkannt, eine solchermaßen gefaltete Folie zu der leichten in-situ Herstellung von Laminaten zu verwenden.

Als Material für die Trennfolie ist grundsätzlich jedes silikonisierbare flexible Material geeignet. Um eine absolute Undurchlässigkeit auch für sehr kleine Moleküle sicherzustellen, kann auch eine Polymer-Metall-Verbundfolie verwendet werden.

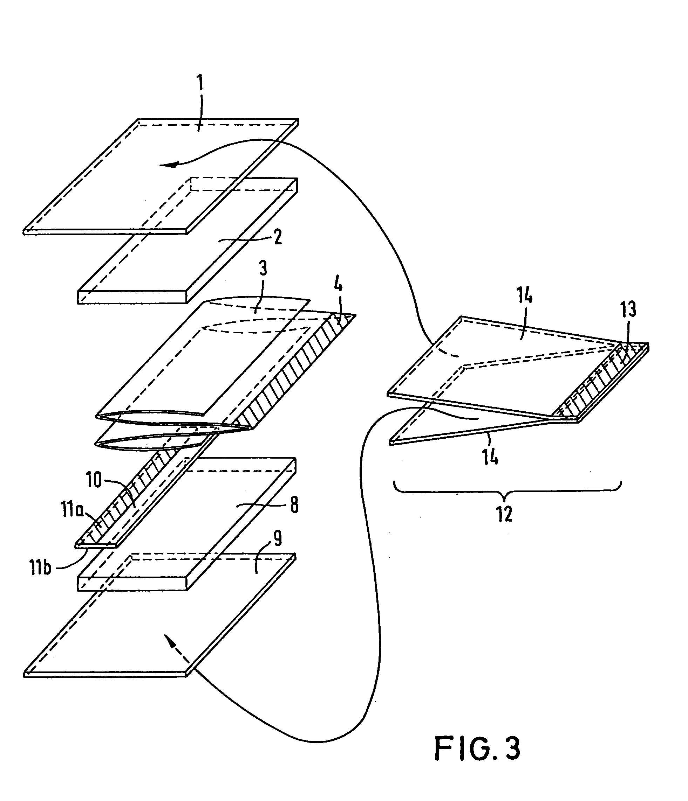

In Fig. 3 ist ein prinzipiell gleich aufgebautes System gezeigt, bei dem allerdings die Lochmaske (5) (Fig. 1) auf einen Streifen (10) reduziert ist, dessen obere Oberfläche (11a) mit dem oberen Reservoirteil (2), und dessen untere Oberfläche (11b) mit dem unteren Reservoirteil in Verbindung steht. Dieser Systemaufbau hat den Vorteil, daß die Kontaktfläche zwischen den beiden zu vereinigenden Reservoirteilen (2) und (8) besonders groß ist, und den der leichteren Herstellbarkeit. Wird eine besonders flexible Trägerfolie (1) verwendet, so kann bei diesem System bei sehr ungeschicktem Hantieren eventuell ein seitlich versetztes Aufeinanderlaminieren der Reservoirteile (2) und (8) erfolgen. Um dies zu vermeiden, findet die Laminierungshilfe (12) Verwendung. Das System liegt zwischen den Flächen (14) der Laminierungshilfe (12), und die herausziehbare Trennfolie (3) ist auf der Fläche (13) mit der Laminierungshilfe verbunden. Durch Herausziehen des Systems aus der Laminierungshilfe ist das Gesamtlaminat hergestellt, wobei ein leichter Druck auf die Flächen (14) den Laminierungsprozeß unterstützt, und ein seitlich versetztes Laminieren wirkungsvoll verhindert.

Anstelle einer Lochmaske (5, 10) können die Reservoirteile (2, 8) auch über eine lösbare Verbindung zwischen der Rückschicht bzw. Trägerfolie (1) und der entfernbaren Schutzfolie (9) in Kontakt stehen, wobei Rückschicht (1) und Schutzschicht (9) mindest auf einer Seite die Reservoirflächen (2, 8) überragen.

Da diese Laminierungshilfe eine Applikation des Systems auf die Haut ohne vorheriges Herstellen des Gesamtlaminats unmöglich macht, ist sie, um ein versehentliches Vergessen des Laminierungsprozesses zu vermeiden, in diesem Sinne auch in Verbindung mit dem in Fig. 1 gezeigten Systemaufbau sinnvoll verwendbar.

Damit ist aber auch ein Vorteil des Systemaufbaus gemäß Fig. 1 angesprochen. Dieser Aufbau erlaubt nämlich im Gegensatz zu allen bisher beschriebenen Systemen auch nach einer beliebig langen Zeit nach dem Aufkleben auf die Haut die Herstellung des Gesamtlaminats.

In Fig. 4 ist ein System gezeigt, bei dem die Zwischenschicht durch einen gasgefüllten Spalt gebildet wird. Auf dem Boden einer auf der Innenseite - wenn erforderlich - silikonisierten Tiefziehform (15) befindet sich der Reservoirteil (16), der nach Applikation Kontakt mit der Haut hat. Der obere Reservoirteil (17) befindet sich auf der Trägerfolie (18), die für die Inhaltsstoffe von (17) undurchlässig ist. Die Trägerfolie (18) ist auf der Fläche (19) mit der Tiefziehform (15) verklebt. Der Reservoirteil (17) kann aber flächenmäßig auch die gleiche Ausdehnung wie die Trägerfolie (18) haben, so daß (17) auf der Fläche (19) mit der Tiefziehform Kontakt hat. Vor Applikation wird durch mechanischen Druck auf die Tiefziehform der Kontakt zwischen den beiden Laminatteilen (16) und (17) hergestellt und damit das Gesamtsystem erhalten. Zur Applikation auf die Haut ist es jetzt nur noch nötig, die Tiefziehform, die hier die Rolle der Abziehfolie (9) (Fig. 3 und 4) übernimmt, zu entfernen.

Für alle Systemaufbauten gilt, daß die zu vereinigenden Reservoirteile des Systems (2) und (8) einen beliebig komplizierten Aufbau besitzen können. Insbesondere der Einbau von zusätzlichen, die Diffusion steuernden Membranen kann sich dabei als nützlich erweisen.

| Bezugszeichenliste | |
| --- | --- |
| Undurchlässige Rückschicht bzw. Trägerfolie | 1, 18 |
| Der Haut nach Applikation abgewandtes oberes Reservoirteil | 2, 17 |
| Herausziehbare Trennfolie | 3 |
| Anfaßstück der Trennfolie | 4 |
| Lochmaske | 5, 10 |
| Kontaktfläche zwischen Lochmaske und oberem Reservoirteil | 6a,11a |
| Kontaktfläche zwischen Lochmaske und unterem Reservoirteil | 6b,11b |
| Aussparung in der Lochmaske | 7 |
| Der Haut nach Applikation zugewandtes unteres Reservoirteil | 8, 16 |
| Ablösbare Schutzfolie | 9 |
| Laminierungshilfe | 12 |
| Verbindungsfläche zwischen Trennfolie (3) und Laminierungshilfe (12) | 13 |
| Andrückflächen der Laminierungshilfe | 14 |
| Innen silikonisierte Tiefziehform | 15 |
| Klebende Flächenverbindung zwischen Tiefziehform (15) und Trägerfolie (18) | 19 |

**Patentansprüche**
**Patentansprüche für folgende Vertragsstaaten : AT, BE, CH, DE, DK, FR, GB, IT, LI, LU, NL, SE**

1. Therapeutisches System zur transdermalen oder transmucosalen Verabreichung von Wirkstoffen, bestehend aus einer undurchlässigen Rückschicht (1), einem zumindest zum Teil wirkstoffhaltigen zweischichtigen, voneinander getrennnte Teile (2) bzw. (8) aufweisenden Reservoir und einer Schutzschicht (9), wobei die beiden Reservoirteile (2) bzw. (8)
   (a) weitgehend parallel übereinander angeordnet sind,
   (b) entweder Wirkstoff, Hilfsstoff oder beides in nicht dem Verteilungsgleichgewicht zwischen therapeutischem System und der Haut entsprechenden Konzentrationen enthalten,
   dadurch gekennzeichnet, daß die beiden Reservoirteile (2) bzw. (8) selbstklebend und durch eine für die Inhaltsstoffe der beiden Reservoirteile undurchlässige entfernbare Zwischenschicht voneinander getrennt sowie, über eine Maske (5) partiell miteinander verklebt sind und die Zwischenschicht (3) zwischen einem Reservoirteil (2) bzw. (8) und der Maske (5) angeordnet und aus einer gefalteten, herausziehbaren Folie gebildet ist, die die Flächen der Reservoirteile mindestens teilweise seitlich überragt.

2. Therapeutisches System zur transdermalen oder transmucosalen Verabreichung von Wirkstoffen, bestehend aus einer undurchlässigen Rückschicht (1), einem zumindest zum Teil wirkstoffhaltigen zweischichtigen, voneinander getrennte Teile (2) bzw. (8) aufweisenden Reservoir und einer undurchlässigen und ablösbaren Schutzschicht (9), wobei die beiden Reservoirteile (2) bzw. (8)
   (a) weitgehend parallel übereinander angeordnet sind,
   (b) entweder Wirkstoff, Hilfsstoff oder beides in nicht dem Verteilungsgleichgewicht zwischen therapeutischem System und der Haut entsprechenden Konzentrationen enthalten,
   dadurch gekennzeichnet, daß die Reservoirteile durch eine für die Inhaltsstoffe der beiden Reservoirteile undurchlässige, entfernbare Zwischenschicht (3) voneinander getrennt sowie selbstklebend sind und die Zwischenschicht durch einen gasgefüllten Spalt gebildet ist.

3. Therapeutisches System nach Anspruch 1, dadurch gekennzeichnet, daß die Zwischenschicht (3) aus silikonisiertem, flexiblem Material besteht.

4

**4.** Therapeutisches System nach Anspruch 1, dadurch gekennzeichnet, daß die Zwischenschicht (3) aus einer PolymerMetall-Verbundfolie besteht.

**5.** Therapeutisches System nach Anspruch 1, dadurch gekennzeichnet, daß die Maske als ein die Längskanten der beiden Reservoirteile (2) bzw. (8) verbindender Streifen (10) ausgebildet ist.

**6.** Verwendung des therapeutischen Systems nach Ansprüchen 1 bis 5, dadurch gekennzeichnet, daß das System durch Vereinigung der beiden Reservoirteile (2) bzw. (8) unter Entfernung der undurchlässigen Zwischenschicht (3) aktiviert wird.

**7.** Verwendung des therapeutischen Systems nach Anspruch 1, dadurch gekennzeichnet, daß die die undurchlässige Zwischenschicht bildende gefaltete Folie (3) durch Herausziehen entfernt wird.

**8.** Verwendung des therapeutischen Systems nach Anspruch 2, dadurch gekennzeichnet, daß die beiden Reservoirteile (2) bzw. (8) unter Anwendung von Druck senkrecht zur Zwischenschicht vereinigt werden.

**Patentansprüche für folgende Vertragsstaaten : ES, GR**

**1.** Verfahren zur Herstellung eines therapeutischen Systems zur transdermalen oder transmucosalen Verabreichung von Wirkstoffen, das aus einer undurchlässigen Rückschicht (1), einem mindestens zum Teil wirkstoffhaltigen zweischichtigen, voneinander getrennte Teile aufweisenden Reservoir (2,8) und einer Schutzschicht (9) besteht, wobei die beiden Reservoirteile (2,8)
    (a) weitgehend parallel übereinander angeordnet sind,
    (b) entweder Wirkstoff, Hilfsstoff oder beides in nicht dem Verteilungsgleichgewicht zwischen therapeutischem System und der Haut entsprechenden Konzentrationen enthalten,
dadurch gekennzeichnet, daß die beiden selbstklebenden Reservoirteile (2, 8) durch eine für die Inhaltsstoffe der beiden Reservoirteile undurchlässige entfernbare Zwischenschicht voneinander getrennt sind und über eine Maske (5) partiell miteinander verklebt werden, wobei eine Zwischenschicht (3) zwischen einem Reservoirteil (2) bzw. (8) und der Maske (5) angeordnet und aus einer gefalteteten, herausziehbaren Folie gebildet wird, die die Flächen der Reservoirteile (2) bzw. (8) mindestens teilweise seitlich überragt.

**2.** Verfahren zur Herstellung eines therapeutischen Systems zur transdermalen oder transmucosalen Verabreichung von Wirkstoffen, das aus einer undurchlässigen Rückschicht (1), einem mindestens zum Teil wirkstoffhaltigen zweischichtigen, voneinander getrennte Teile (2) bzw. (8) aufweisenden Reservoir und einer undurchlässigen und ablösbaren Schutzschicht (9) besteht, wobei die beiden Reservoirteile (2) bzw. (8)
    (a) weitgehend parellel übereinander angeordnet werden,
    (b) entweder Wirkstoff, Hilfsstoff oder beides in nicht dem Verteilungsgleichgewicht zwischen therapeutischem System und der Haut entsprechenden Konzentrationen enthalten,
dadurch gekennzeichnet, daß die selbstklebenden Reservoirteile durch eine für die Inhaltsstoffe der beiden Reservoirteile undurchlässige, entfernbare Zwischenschicht (3) voneinander getrennt und partiell direkt miteinander verklebt werden, wobei die Zwischenschicht durch einen gasgefüllten Spalt gebildet wird.

**3.** Verfahren nach Anspruch 1, dadurch gekennzeichnet, daß als Zwischenschicht (3) ein silikonisiertes, flexibles Material verwendet wird.

**4.** Verfahren nach Anspruch 1, dadurch gekennzeichnet, daß als Zwischenschicht (3) eine polymer-Metall-Verbundfolie verwendet wird.

**5.** Verfahren nach Anspruch 1, dadurch gekennzeichnet, daß die beiden Reservoirteile (2) bzw. (8) über eine Maske (5) partiell miteinander verklebt werden und diese als ein die Längskanten der beiden Reservoirteile verbindender Streifen (10) ausgebildet wird.

**6.** Verwendung des therapeutischen Systems nach Ansprüchen 1 bis 5, dadurch gekennzeichnet, daß das System durch Vereinigung der beiden Reservoirteile (2) bzw. (8) unter Entfernung der undurchlässigen Zwischenschicht (3) aktiviert wird.

**7.** Verwendung des therapeutischen Systems nach Anspruch 1, dadurch gekennzeichnet, daß die die undurchlässige Zwischenschicht (3) bildende Folie durch Herausziehen entfernt wird.

**8.** Verwendung des therapeutischen Systems nach Anspruch 2, dadurch gekennzeichnet, daß die beiden Reservoirteile (2) bzw. (8) unter Anwendung von Druck senkrecht zur Zwischenschicht (3) vereinigt werden.

**Claims**
**Claims for the following Contracting States : AT, BE, CH, DE, DK, FR, GB, IT, LI, LU, NL, SE**

**1.** Therapeutic system for the transdermal or transmucous administration of active substances comprising an impermeable backing layer (1), a reservoir which at least partially contains active substance, which is double-layered and which comprises separate members (2) and (8), and a protective layer (9), wherein the two reservoir members (2) and (8)
   (a) are positioned substantially parallel on top of one another,
   (b) either comprise active substance or auxiliary agents or both at concentrations not corresponding to the distribution equilibrium between the therapeutic system and the skin,
   characterized in that both reservoir members (2) and (8) are self-adhesive, are separated from each other by a removable intermediate layer which is impermeable to the substances contained in said two reservoir members, and are partially adhesively bonded via a mask (5), that the intermediate layer (3) is positioned between one reservoir member (2) or (8), respectively, and said mask (5) and is formed of a folded foil which can be removed by pulling it out and which at least partially extends laterally beyond the surfaces of the reservoir members.

**2.** Therapeutic system for the transdermal or transmucous administration of active substances comprising an impermeable backing layer (1), a reservoir which at least partially contains active substance, which is double-layered and which comprises separate members (2) and (8), and an impermeable and removable protective layer (9), wherein the two reservoir members (2) and (8)
   (a) are positioned substantially parallel on top of one another,
   (b) either contain active substance or auxiliary agents or both at concentrations not corresponding to the distribution equilibrium between the therapeutic system and the skin,
   characterized in that the reservoir members are self-adhesive, are separated from each other by a removable intermediate layer (3) which is impermeable to the substances contained in said two reservoir members, and that the intermediate layer is formed of a slit filled with gas.

**3.** The therapeutic system according to claim 1, characterized in that the intermediate layer (3) consists of a siliconized, flexible material.

**4.** The therapeutic system according to claim 1, characterized in that the intermediate layer (3) consists of a polymer-metal-composite foil.

**5.** The therapeutic system according to claim 1, characterized in that the mask is formed as a strip (10) joining the longitudinal edges of the two reservoir members (2) and (8).

**6.** The use of the therapeutic system according to claims 1 to 5, characterized in that the system is activated by combining both reservoir members (2) and (8) by removal of the impermeable intermediate layer (3).

**7.** The use of the therapeutic system according to claim 1, characterized in that the folded foil (3) forming the impermeable intermediate layer is removed by pulling it out.

**8.** The use of the therapeutic system according to claim 2, characterized in that the two reservoir members (2) and (8) are combined by application of pressure vertically to the intermediate layer.

**Claims for the following Contracting States : ES, GR**

**1.** Process for the production of a therapeutic system for the transdermal or transmucous administration of active substances comprising an impermeable backing layer (1), a reservoir which at least partially

6

contains active substance, which is double-layered and which comprises separate members, and a protective layer (9), wherein the two reservoir members (2, 8)

(a) are positioned substantially parallel on top of one another,

(b) either comprise active substance or auxiliary agents or both at concentrations not corresponding to the distribution equilibrium between the therapeutic system and the skin,

characterized in that said two self-adhesive reservoir members (2, 8), are separated from each other by a removable intermediate layer which is impermeable to the substances contained in said two reservoir members, and are partially adhesively bonded via a mask (5), whereby an intermediate layer (3) is positioned between one reservoir member (2) or (8), respectively, and said mask (5) and is formed of a folded foil which can be removed by pulling it out and which at least partially extends laterally beyond the surfaces of the reservoir members (2) and (8).

2. Process for the production of a therapeutic system for the transdermal or transmucous administration of active substances comprising an impermeable backing layer (1), a reservoir which at least partially contains active substance, which is double-layered and which comprises separate members (2) and (8), and an impermeable and removable protective layer (9), wherein the two reservoir members (2) and (8)

(a) are positioned substantially parallel on top of one another,

(b) either contain active substance or auxiliary agents or both at concentrations not corresponding to the distribution equilibrium between the therapeutic system and the skin,

characterized in that the self-adhesive reservoir members are separated from each other by a removable intermediate layer (3) which is impermeable to the substances contained in said two reservoir members, and are partially and directly adhesively bonded, whereby the intermediate layer is formed of a slit filled with gas.

3. The process according to claim 1, characterized in that as intermediate layer (3) a siliconized, flexible material is used.

4. The process according to claim 1, characterized in that as intermediate layer (3) a polymer-metal-composite foil is used.

5. The process according to claim 1, characterized in that the two reservoir members (2) and (8) are partially bonded via a mask (5) and that said mask is formed as a strip (10) joining the longitudinal edges of the two reservoir members.

6. The use of the therapeutic system according to claims 1 to 5, characterized in that the system is activated by combining both reservoir members (2) and (8) by removal of the impermeable intermediate layer (3).

7. The use of the therapeutic system according to claim 1, characterized in that the folded foil forming the impermeable intermediate layer (3) is removed by pulling it out.

8. The use of the therapeutic system according to claim 2, characterized in that the two reservoir members (2) and (8) are combined by application of pressure vertically to the intermediate layer (3).

**Revendications**

**Revendications pour les Etats contractants suivants : AT, BE, CH, DE, DK, FR, GB, IT, LI, LU, NL, SE**

1. Système thérapeutique destiné à l'administration transdermique ou transmucosale de principes actifs, qui se compose d'une couche dorsale (1) imperméable, d'un réservoir présentant des parties mutuellement séparées (2) et (8) bicouches et contenant partiellement du principe actif et d'une couche de protection (9), où les deux parties de réservoir (2) et (8)

(a) sont agencées sensiblement parallèlement l'une par-dessus l'autre,

(b) contiennent du principe actif, une substance auxiliaire ou du principe actif et de la substance auxiliaire à la fois en concentrations ne correspondant pas à l'équilibre de répartition entre le système thérapeutique et la peau,

caractérisé en ce que les deux parties (2) et (8) du réservoir sont autocollantes et mutuellement séparées par une couche intermédiaire éliminable et imperméable aux substances contenues dans les deux parties du réservoir et sont aussi partiellement mutuellement collées par l'intermédiaire d'un masque, et la couche intermédiaire (3) est agencée entre une partie (2) et (8) et le masque (5) et est formée d'une feuille pliée, arrachable, qui dépasse latéralement au moins partiellement les surfaces des parties du réservoir.

2.  Système thérapeutique destiné à l'administration transdermique ou transmucosale de principes actifs, qui se compose d'une couche dorsale (1) imperméable, d'un réservoir présentant des parties mutuellement séparées (2) et (8) bicouches et contenant partiellement du principe actif et d'une couche de protection (9) imperméable et détachable, où les deux parties de réservoir (2) et (8)

(a) sont agencées sensiblement parallèlement l'une par-dessus l'autre,
(b) contiennent du principe actif, une substance auxiliaire ou du principe actif et de la substance auxiliaire à la fois en concentrations ne correspondant pas à l'équilibre de répartition entre le système thérapeutique et la peau,

caractérisé en ce que les parties du réservoir sont autocollantes, comme aussi mutuellement séparées par une couche intermédiaire (3) éliminable, imperméable aux substances contenues par les deux parties du réservoir et la couche intermédiaire est formée par une fente remplie de gaz.

3.  Système thérapeutique suivant la revendication 1, caractérisé en ce que la couche intermédiaire (3) est constituée par un matériau souple et siliconé.

4.  Système thérapeutique suivant la revendication 1, caractérisé en ce que la couche intermédiaire est constituée d'une feuille composite de polymère-métal.

5.  Système thérapeutique suivant la revendication 1, caractérisé en ce que le masque est réalisé sous forme d'une bandelette (10) unissant les côtés longitudinaux des deux parties du réservoir (2) et (a).

6.  Utilisation du système thérapeutique suivant l'une quelconque des revendications 1 à 5, caractérisée en ce que l'on active le système par la réunion ou l'assemblage des deux parties du réservoir (2) et (8) en éliminant la couche intermédiaire imperméable (3).

7.  Utilisation du système thérapeutique suivant la revendication 1, caractérisée en ce que la feuille pliée (3) formant la couche intermédiaire imperméable est éliminée par arrachement.

8.  Utilisation du système thérapeutique suivant la revendication 2, caractérisée en ce que l'on réunit ou assemble les deux parties du réservoir (2) et (8) par l'application d'une pression verticale sur la couche intermédiaire.

**Revendications pour le Etat contractants suivants : ES, GR**

1.  Procédé de préparation d'un système thérapeutique destiné à l'administration transdermique ou transmucosale de principes actifs, qui se compose d'une couche dorsale (1) imperméable, d'un réservoir présentant des parties mutuellement séparées (2, 8) bicouches et contenant partiellement du principe actif et d'une couche de protection (9), où les deux parties du réservoir (2, 8)

(a) sont agencées sensiblement parallèlement l'une par-dessus l'autre,
(b) contiennent du principe actif, une substance auxiliaire ou du principe actif et de la substance auxiliaire à la fois en concentrations ne correspondant pas à l'équilibre de répartition entre le système thérapeutique et la peau,

caractérisé en ce que les deux parties du réservoir (2, 8) autocollantes sont mutuellement séparées par une couche intermédiaire éliminable et imperméable aux substances contenues dans les deux parties du réservoir et sont partiellement mutuellement collées par-dessus un masque (5), où une couche intermédiaire (3) est agencée entre une partie du réservoir (2) et (a) et le masque (5) et cette couche intermédiaire est formée d'une feuille arrachable et pliée, qui dépasse au moins partiellement latéralement des surfaces des parties du réservoir (2) et (8).

2.  Procédé de préparation d'un système thérapeutique destiné à l'administration transdermique ou transmucosale de principes actifs, qui se compose d'une couche dorsale (1) imperméable, d'un réservoir présentant des parties mutuellement séparées (2) et (8) bicouches et contenant partiellement du principe actif et d'une couche de protection (9) imperméable et détachable, où les deux parties du réservoir (2) et (8)

(a) sont agencées sensiblement parallèlement l'une par-dessus l'autre,

(b) contiennent du principe actif, une substance auxiliaire ou du principe actif et de la substance auxiliaire à la fois en concentrations ne correspondant pas à l'équilibre de répartition entre le système thérapeutique et la peau,

caractérisé en ce que les parties du réservoir autocollantes sont mutuellement séparées par une couche intermédiaire (3) éliminable et imperméable aux substances contenues dans les deux parties du réservoir et sont partiellement directement mutuellement collées, où la couche intermédiaire est formée par une fente remplie de gaz.

3. Procédé suivant la revendication 1, caractérisé en ce que l'on utilise un matériau souple et siliconé à titre de couche intermédiaire (3).

4. Procédé suivant la revendication 1, caractérisé en ce que l'on utilise une feuille composite de polymère-métal à titre de couche intermédiaire (3).

5. Procédé suivant la revendication 1, caractérisé en ce que les deux parties du réservoir (2) et (8) sont partiellement mutuellement collées par-dessus un masque (5) et en ce que ce masque est formé par une bandelette (10) liant ou unissant les côtés longitudinaux des deux parties du réservoir.

6. Utilisation du système thérapeutique suivant l'une quelconque des revendications 1 à 5, caractérisée en ce que l'on active le système par la réunion ou l'assemblage des deux parties du réservoir (2) et (8) en éliminant la couche intermédiaire imperméable (3).

7. Utilisation du système thérapeutique suivant la revendication 1, caractérisée en ce que la feuille formant la couche intermédiaire (3) imperméable est éliminée par arrachement.

8. Utilisation du système thérapeutique suivant la revendication 2, caractérisée en ce que l'on réunit ou assemble les deux parties du réservoir (2) et (8) par l'application d'une pression verticale sur la couche intermédiaire.

# FIG.1

FIG.2

FIG.4

EP 0 382 129 B1

FIG.3